# EUROPEAN PATENT SPECIFICATION

(11) **EP 2 923 698 B1**
(45) Date of publication and mention of the grant of the patent: **28.04.2021**
(21) Application number: 13857345.6
(22) Date of filing: 22.11.2013
(51) Int. Cl.: A61K 31/366, A61K 31/22, A61K 31/505, A61K 31/40, A61K 31/405, A61K 31/4418, A61K 31/47, A61K 9/08, A61P 1/16, A61P 3/04, A61P 3/06, A61P 3/10, A61P 9/10, A61P 9/12, A61K 9/00

(54) **STATIN COMPOUND FOR USE IN TREATING OBESITY, HYPERTENSION AND HYPERLIPAEMIA BY INTRAOSSEOUS ADMINISTRATION**
STATINVERBINDUNG ZUR VERWENDUNG BEI DER BEHANDLUNG VON ADIPOSITAS, HYPERTONIE UND HYPERLIPÄMIE DURCH INTRA-OSSALE VERABREICHUNG
COMPOSÉ DE TYPE STATINE POUR UTILISATION DANS LE TRAITEMENT DE L'OBÉSITÉ, DE L'HYPERTENSION ET DE L'HYPERLIPIDÉMIE PAR ADMINISTRATION INTRAOSSEUSE

(30) Priority: 23.11.2012 CN 201210512630; 18.09.2013 CN 201310426327
(43) Date of publication of application: 30.09.2015
(73) Proprietor: Peking University Third Hospital, Beijing 100191 (CN)
(72) Inventor: SONG, Chunli, Beijing 100191 (CN); YANG, Ning, Beijing 100191 (CN); CUI, Yueyi, Beijing 100191 (CN); XU, Yingsheng, Beijing 100191 (CN)
(74) Representative: J A Kemp LLP
(86) International application number: PCT/CN2013/001429
(87) International publication number: WO 2014/079151

(56) References cited:
- EP-A1- 2 057 987
- EP-B1- 2 787 993
- WO-A1-2013/088161
- CN-A- 101 511 348
- CN-A- 101 658 677
- CN-A- 101 658 677
- CN-A- 103 127 505
- CN-A- 103 127 505
- US-A1- 2005 182 125
- ZHOU, DONGFENG ET AL. JOURNAL OF DENTAL PREVENTION AND TREATMENT vol. 13, no. 4, 2005, pages 258 - 261, XP008179492

## Description

### Field of the invention

The present disclosure relates to medicine technology, more specifically, it relates to a new use of HMG-CoA reductase inhibitor (a statin compound) locally used for the prevention of obesity, hypertension, hyperlipidemia, artheroslerosis and fatty liver, the treatment of obesity, hypertension, hyperlipidemia, atherosclerosis, cardiovascular or cerebrovascular diseases which are coronary heart disease and stroke, and fatty liver, and for improving lipid metabolism. The present disclosure also relates to a locally used composition. The present invention relates to a statin compound (HMG CoA reductase inhibitor), or its pharmaceutically acceptable salt or a pharmaceutical composition containing a statin compound and/or its pharmaceutically acceptable salt for use in the prevention, treatment or therapeutic improvement, by local single intraosseous administration, of the diseases as defined in the claims.

### Background of the invention

The prevalence and incidence of obesity is increasing dramatically in both developed and developing countries, and the age of patients suffering from obesity has decreased. It has become a global epidemic of serious health effects. Obesity not only affects the daily life of patients, induces psychological problems, and probably causes social discrimination; the most serious consequence is that obesity causes many diseases, such as type 2 diabetes, coronary heart diseases, hypertension, hyperlipidemia, cholecystitis, arthritis and some cancers. About 80% of patients with obesity have one, and 40% of patients with obesity have two, of the diseases mentioned above (Xu Manyin editor, diabetes, second edition, Shanghai science and technology publishing house).

Obesity may increase the burden on the tissues and organs. The most common diseases caused by obesity are fatty liver, hyperlipidemia, hypertension, coronary heart disease, type 2 diabetes, et al. Obesity is the prevention target of many diseases, and body weight control is also important for diabetes early prevention.

The risk of hypertension and hyperlipidemia is much higher in patients with obesity than those of normal body weight, and hypertension and hyperlipidemia are important causes of arteriosclerosis. The prevalence of coronary heart disease (CHD) is therefore raised in patients with obesity. As the excess energy intake is converted into fatty acid, too much fatty acid is transported to the liver. The excess fatty acid can't be digested by the liver cells, which causes fatty infiltration of liver cells and finally leads to fatty liver. Severe fatty liver can turn into liver cirrhosis.

According to a report from the American National Association of Diabetes, in light, moderate and severe obesity, the risk of developing type 2 diabetes respectively is 2, 5, and 10 times the risk with normal body weight (Xu Manyin editor, diabetes, second edition, Shanghai science and technology publishing house).

The incidence of diabetes in the world has been increasing year by year. Diabetes has become the world's third chronic disease after tumor and cardio-cerebrovascular diseases, which cause serious damage to human health. In recent years the incidence of diabetes has become higher and higher, and the number of cases has surged significantly. According to statistics, the morbidity in the population over 20 years old in China has reached 9.7%, approaching 100 million people. The multiple complications caused by diabetes have high mortality and morbidity rates, and cause serious damage to social health, result in high medical costs, and are a heavy burden on society. Diabetes is mainly divided into two types. In type I diabetes (insulin dependent, IDDM), insulin secretion is absolutely inadequate and exogenous insulin treatment is needed. In type II diabetes (non-insulin dependent, NIDDM), due to the relative lack of insulin, NIDDM has the feature of fasting hyperglycemia and excessive postprandial plasma glucose levels. In NIDDM, high blood glucose, insulin resistance and insulin secretion deficiency leads to elevated blood glucose.

Diabetes treatments include insulin (injected or inhaled), and oral medications including:
Sulfonylurea (e.g., tolbutamide, glyburide, glipizide, gliclazide, glimepiride and glipizide);
Glinides (e.g., repaglinide and nateglinide);
Biguanide (such as metformin and phenformin);
Insulin synergistic agents (such as rosiglitazone and pioglitazone);
Alpha glycosidase inhibitors (such as acarbose and voglibose).

In previous clinical studies, the effect of statins on diabetes has been inconsistent (Swapnil N. Rajpathak et al., Statin therapy and risk of developing type 2 diabetes: a meta-analysis. Diabetes Care. 2009; 32(10): 1924-1929).

Tan et al. reported the effect of orally administrated atorvastatin on the blood glucose in patients with type 2 diabetes. All the patients were treated with conventional antidiabetics, and the experimental group was supplemented with atorvastatin 10mg/d for 16 weeks. The results showed that the blood glucose in the atorvastatin group was much better than in the control group.

Michiro, Ishikawa, et al. reported that in oral pravastatin and atorvastatin treatment of non-diabetic patients with hypercholesterolemia, oral pravastatin was good for glucose metabolism, but atorvastatin had the opposite effect (DOI: 10.2169/internal medicine.45.1476). Jun Sasaki reviewed the impact of statins on diabetes mellitus and glucose metabolism, and described that orally administrated pravastatin is favourable for sugar metabolism, but atorvastatin (10 mg/day) and simvastatin (40 mg/day) have no effect on diabetes. However, orally administrated pravastatin and high dose atorvastatin (80 mg) is detrimental for glucose metabolism (Sasaki J1, Iwashita M, Kono S. Statins: beneficial or adverse for glucose metabolism. J Atheroscler Thromb. 2006 Jun;13(3):123-9.).

On the other hand there are also large amounts of clinical data showing that taking statins increases the incidence of diabetes. Some clinical meta-analysis reported that statins would reduce cardiovascular events, but will slightly increase the risk of diabetes (Statins and risk of incident diabetes: a collaborative meta-analysis of randomised statin trials. Lancet. 2010; 375(9716): 735-742.), (Risk of incident diabetes with intensive-dose compared with moderate-dose statin therapy: a meta-analysis. JAMA. 2011; 305(24): 2556-2564.).

Hypertension, hyperlipidemia, diabetes and obesity are all important causes of atherosclerosis, which causes a great risk of cardiocerebrovascular events. Lipid-lowering and antihypertensive treatments are therefore important strategies to prevent cardiovascular events.

However, without exception, reports conclude that these treatments are based on daily oral statins.

EP 2057987 describes a statin-loaded nanoparticle that allows for local delivery of a statin for therapeutic neovascularization. CN-A-103127505 discloses a statin (HMG-CoA reductase inhibitor) for use in the treatment of non-insulin dependent diabetes (NIDM). The drug is administered topically in a non-reservoir form, most preferably intraosseously.

Statins act as lipid-lowering, inhibiting the key enzyme (HMG-CoA reductase) that is used to lower cholesterol. They have been in safe clinical application for decades, but they require daily use for a long time.

### Summary of the invention

The invention is defined by the claims. Any subject-matter falling outside the scope of the claims is provided for information puposes only. Any references in the description to methods of treatment refer to the compounds, pharmaceutical compositions and medicaments of the present invention for use in a method of treatment of the human or animal body by therapy.

The inventor was surprised to find that a local single application of statin compound (HMG CoA reductase inhibitor) can, for a long time, promote the secretion of insulin, increase insulin sensitivity of peripheral tissue, and lower blood glucose, and can be used in the treatment of diabetes (not part of the claimed invention).

The inventor also surprisingly found that a local single application of statin compound can, for a long time, reduce cholesterol, triglyceride and low density lipoprotein, and improve lipid metabolism. Further, it can reduce body weight, reduce body fat, reduce fatty liver, and can be used in the treatment of obesity, hypertension, hyperlipidemia, fatty liver and used to improve lipid metabolism.

Intraosseous local single use of statin compounds can prevent cardiovascular and cerebrovascular events. Local single intraosseous application of a statin compound can prevent and cure atherosclerosis, coronary heart disease, stroke and other cardiovascular and cerebrovascular diseases.

The purpose of the present invention is to provide a use of statin compounds (HMG CoA reductase inhibitors) as a local single use drug for:
(a) preventing obesity, hypertension, hyperlipidemia, atherosclerosis, or fatty liver;
(b) treating obesity, hypertension, hyperlipidemia, atherosclerosis, cardiovascular or cerebrovascular diseases which are coronary heart disease and stroke, or fatty liver; or
(c) improving lipid metabolism.

Accordingly, the present invention provides a statin compound (HMG CoA reductase inhibitor), or its pharmaceutically acceptable salt or a pharmaceutical composition containing a statin compound and/or its pharmaceutically acceptable salt for use in:
(a) the prevention, by local single intraosseous administration, of obesity, hypertension, hyperlipidemia, atherosclerosis, or fatty liver;
(b) the treatment, by local single intraosseous administration, of obesity, hypertension, hyperlipidemia, atherosclerosis, cardiovascular or cerebrovascular diseases which are coronary heart disease and stroke, or fatty liver; or
(c) the therapeutic improvement, by local single intraosseous administration, of fat metabolism in mammals including human beings;
wherein the dosing interval of the administration is once every 7 days up to 600 days.

The present disclosure also provides a pharmaceutical composition locally administered for:
(a) preventing obesity, hypertension, hyperlipidemia, atherosclerosis, or fatty liver;
(b) treating obesity, hypertension, hyperlipidemia, atherosclerosis, cardiovascular or cerebrovascular diseases which are coronary heart disease and stroke, or fatty liver; or
(c) improving lipid metabolism,
characterized in that it contains a statin compound or its pharmaceutically acceptable salt in a prophylactically or therapeutically effective amount and a pharmaceutically acceptable carrier, dilutent or excipient.

The drug is a non-storage form for local administration. Preferably the drug is an injectable dosage form that is administered skeletally, most preferably intraosseously. The amount of statin compound in the drug unit dosage form ranges from 0.1mg to 1000mg, preferably from 1mg to 500mg, more preferably from 2mg to 200mg, most preferably from 2mg to 10mg.

The statin compound of the present invention may be selected from simvastatin, atorvastatin, fluvastatin, lovastatin, pravastatin, rosuvastatin, pitavastatin, bervastatin, cerivastatin, crilvastatin, dalvastatin, mevasatin or tenivastatin; preferably simvastatin, atorvastatin, fluvastatin, or rosuvastatin. Most preferably, the statin compound is simvastatin.

Said statin compounds also include pharmaceutically acceptable salt forms, which may be selected from hydrochloride, hydrobromide, hydriodate, sulfate, nitrate, phosphate, citrate, mesylate, trifluoroacetate, or acetate salts, or salts of sodium, potassium, magnesium, calcium, or magnesium thereof. For example, calcium atorvastatin calciumsodium atorvastatin, sodium fluvastatin, sodium pravastatin, calcium rosuvastatin, or calcium pitavastatin.

The application also discloses a pharmaceutical composition, which contains a statin compound and a pharmaceutically acceptable carrier, diluent or excipient.

The application also discloses a pharmaceutical composition suitable for intraosseous injection, which contains a statin compound and a pharmaceutically acceptable carrier, diluent or excipient.

This application also discloses a pharmaceutical formulation locally administered for:
(a) preventing obesity, hypertension, hyperlipidemia, atherosclerosis, or fatty liver;
(b) treating obesity, hypertension, hyperlipidemia, atherosclerosis, cardiovascular or cerebrovascular diseases which are coronary heart disease and stroke, or fatty liver; or
(c) improving lipid metabolism,
characterized in that it contains an effective amount of statin compound and pharmaceutically acceptable carriers, diluents or excipients. Effective amount means an amount wherein after local administration, the formulation can be effective in preventing obesity, hypertension, hyperlipidemia, fatty liver and atherosclerosis, or treating obesity, hypertension, hyperlipidemia, fatty liver, atherosclerosis and cardiovascular and cerebrovascular diseases which are coronary heart disease and stroke. Preferably, the formulation is an injectable dosage form suitable for intraosseous administration.

This application further discloses a method of preparing a pharmaceutical composition, comprising a procedure of mixing a statin compound in a therapeutically effective amount with a pharmaceutically acceptable carrier, diluent or excipient. For example, dissolving or suspending the statin compound in a therapeutically effective amount in said pharmaceutically acceptable carrier, diluent or excipient.

This application also discloses a method of preparing a pharmaceutical formulation, characterized by mixing a statin compound in a therapeutically effective amount with a pharmaceutically acceptable carrier, diluent or excipient to form a pharmaceutical formulation that is to be locally administered; preferably, to form an injectable dosage form suitable for intraosseous administration.

In a specific embodiment of the present invention, the local statin drug (or composition) is administered preferably via injection into bone, and the local statin drugs described in the present invention can exist in the form of injectable dosage forms. The injectable dosage forms include, but are not limited to, injectable solutions, injectable suspension liquids, injectable emulsions, injectable gels, injectable solid forms, or their slow or controlled release forms. Here, the injectable solid form refers to a form that is mixed with a solvent such as water, normal saline or a glucose solution when it is used to make it feasible for intraosseous injection.

In a specific embodiment of the present invention, the local statin drugs (or composition) comprise a statin compound or its pharmaceutically acceptable salt and pharmaceutically acceptable carrier, diluent or excipient. Here, said pharmaceutical acceptable carrier, diluent or excipient can be selected from at least one of an optional water-soluble solvent or oily solvent, dispersing agent, isotonic agent, preservative, solubilizer or stabilizers. The water-soluble solvent can be selected from distilled water, normal saline, Ringer's solution or phosphate buffer (PBS). The oil soluble solvent can be selected from vegetable oil, such as olive oil, castor oil, sesame oil, cottonseed oil or corn oil. The dispersing agent can be selected from tween 20 or tween 80, polyethylene glycol, carboxy methyl cellulose, and/or sodium alginate. The isotonic agent can be selected from sodium chloride, glycerol, sorbic alcohol, or glucose. The solubilizer can be selected from sodium salicylic acid, poloxamer or sodium acetate. The preservative may be selected from methyl paraben, propyl paraben, benzyl alcohol, chlorobutanol, sodium benzoate, or phenol. The stabilizer may be selected from albumin, such as human serum albumin, bovine serum albumin, etc. Preferably, said pharmaceutically acceptable carrier, diluent or excipient can also be selected from biodegradable materials, such as polylactide, poly-l-lactide-glycolide, polyaspartic acid, and so on.

For a person skilled in the art, the statin local medicament (or composition) of the present invention can be prepared through known preparation techniques. For example, the statin compound or its pharmaceutically acceptable salt together with a dispersing agent, and/or isotonic agent, and/or preservative, and/or solubilizer and/or stabilizers are dissolved, suspended or emulsified in a water-soluble solvent or oil-soluble solvent (Remington: the science and practice of pharmacy, 21st edition, 2005, Lippincott Williams).

The dosing interval of the administration of the statin local composition to a mammal is once every 7 days up to 600 days, preferably 10 to 500 days at a time, more preferably 20 to 400 days at a time, most preferably 30 to 300 days at a time. In terms of the administration of the statin local composition to a mammal, a single dose of statin compound is from 0.1mg to 1000mg, preferably from 1mg to 500mg, more preferably from 2mg to 200mg. A clinician can adjust or modify the frequency and dose of administration according to the needs of the clinical effect, under the guidance of the present invention.

In an aspect of the present disclosure (not part of the claimed invention), the disclosure provides a local composition for the treatment of diabetes, wherein a statin compound or its pharmaceutically acceptable salt is present in a pharmacologically effective amount from 0.1mg to 1000mg, preferably from 1mg to 500mg, more preferably from 2mg to 200mg, most preferably from 2mg to 10mg. In addition, the dosing interval of the administration of the statin local composition to a mammal is once every 7 days up to 600 days, preferably 10 to 500 days at a time, more preferably 20 to 400 days, most preferably 30 to 300 days at a time. A person skilled in the art can adjust or modify the frequency and dosage of local administration based on the clinical therapeutic effect requirement.

In a specific embodiment of the present invention, the local statin drug (or composition) is administered preferably via injection into bone or embedding in bone. Here, the bones can be chosen from, but are not limited to, the ilium, distal radius, phalanx, tibia, vertebral body or any other bone which is convenient for local drug delivery, preferably bones with a bone marrow cavity therein.

In an embodiment of the invention, preferably a mammal is a human being.

Experiments show that single, local administration of the statin composition of the invention into a bone containing a bone marrow cavity can significantly induce serum insulin levels, increase insulin sensitivity, and obviously reduce the blood glucose of diabetic mammals. More importantly, a single injection can have a long curative effect, avoiding the current daily diabetes treatments such as everyday oral administration or subcutaneous insulin injection. This can enhance therapeutic compliance and convenience. Most importantly, a single local injection promotes endogenous insulin secretion and enhances insulin sensitivity. It is much better than the existing treatments for diabetes.

Experiments also show that the single intraosseous administration of the statin compound of the present invention can significantly reduce low-density lipoprotein (LDL), cholesterol, and triglyceride levels, and obviously reduce weight, reduce body fat, and reduce fatty liver. A single injection can have a long curative effect, avoiding the current daily treatments of obesity and lipid metabolism which need administration every day. The invention can enhance therapeutic compliance and convenience. These effects are independent of liver HMG CoA reductase, showing a better therapeutic effect and a longer lasting effect but with lower doses required.

### Brief description of the drawings

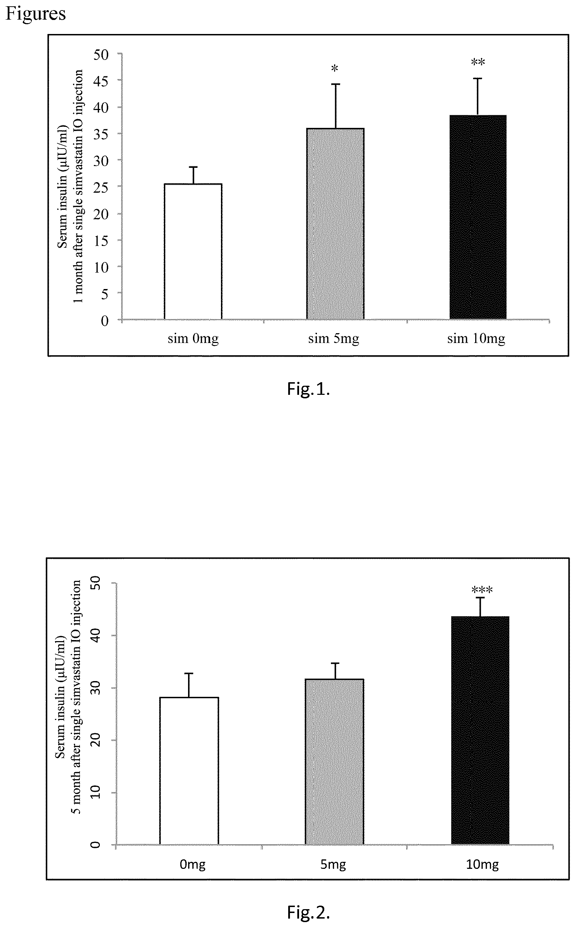

Figure 1 shows the changes of serum insulin levels one month after a local, single injection of simvastatin into the femoral marrow cavity in ovariectomized rats.
   3 months after ovariectomy, simvastatin (0, 5, 10mg) (formula of example 1 of the present invention) was injected into the right femoral medullary cavity of the ovariectomized rats. 1 month later, the rats were euthanized by excessive anesthesia, the serum was collected, and the serum insulin levels were detected. Results show that the serum insulin level is significantly increased (*<0.05 and **p<0.01, compared with the control group).
Figure 2 shows the changes of serum insulin levels five months after a local, single injection of simvastatin into the femoral marrow cavity in ovariectomized rats.
   3 months after ovariectomy, simvastatin (0, 5, 10mg) (formula of example 1 of the present invention) was injected into the right femoral medullary cavity of the ovariectomized rats. 5 months later, the rats were euthanized by excessive anesthesia, the serum was collected, and the serum insulin level were detected. Results show that the serum insulin level is significantly increased (**p<0.01, compared with the control group).

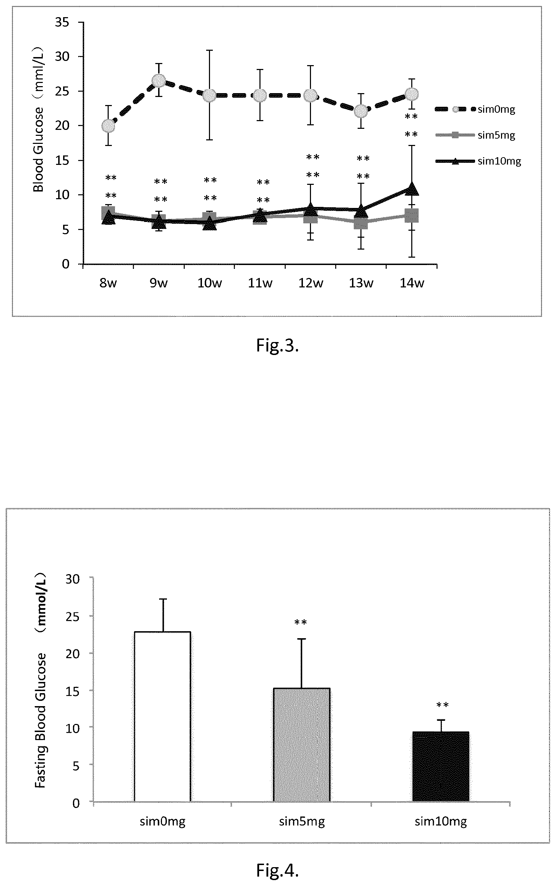

Figure 3 shows the fasting blood glucose changes 8-14 weeks after a local, single injection of simvastatin into the femoral marrow cavity in diabetic rats.
   The fasting blood glucose was detected 8 weeks to 14 weeks after a single intraosseous injection of simvastatin (see formula of example 1) into the diabetic rat's femoral marrow cavity. At the same time, blood was collected via the tail after 12 h of fasting, and a glucose meter measured the blood glucose change. The results show that a single local intraosseous injection of simvastatin significantly reduced fasting blood glucose, and this effect lasted at least 14 weeks.
Figure 4 shows the fasting blood glucose changes 15 weeks after a local, single injection of simvastatin into the femoral marrow cavity in diabetic rats.
   The fasting blood glucose was detected 15 weeks after a single intraosseous injection of simvastatin (see formula of example 1) into the diabetic rat's femoral marrow cavity. At the same time, blood was collected via the abdominal aorta after 8h of fasting, and a glucose meter measured the blood glucose change. The results show that a single local intraosseous injection of simvastatin significantly reduced fasting blood glucose, and this effect lasted at least 15 weeks.
Figure 5 shows the result of a glucose tolerance test (GTT) 11 weeks after a local, single injection of simvastatin (see formula of example 1) into the femoral marrow cavity in diabetic rats.
   According to the standard protocol, after fasting for 12h, the rats received an intraperitoneal injection of glucose (2g/kg), and a GTT test was conducted by blood glucose meter.
Figure 6 shows the result of insulin tolerance test (ITT) 11 weeks after a local, single injection of simvastatin (see formula of example 1) into the femoral marrow cavity in diabetic rats.
   According to the standard protocol, after fasting for 12h, the rats received an intraperitoneal injection of neutral insulin (1U/kg), and a ITT test was conducted by blood glucose meter.
Figure 7 shows the result of a glucose-stimulated insulin secretion test (GSIS) 14 weeks after a local, single injection of simvastatin (see formula of example 1) into the femoral marrow cavity in diabetic rats.
   According to the standard protocol, after fasting for 12h, the rats received an intraperitoneal injection of glucose (3g/kg, China otsuka pharmaceutical co., LTD), blood was gathered from the tail vein of rats, and the insulin levels were detected with trace determination of insulin assay Kit.

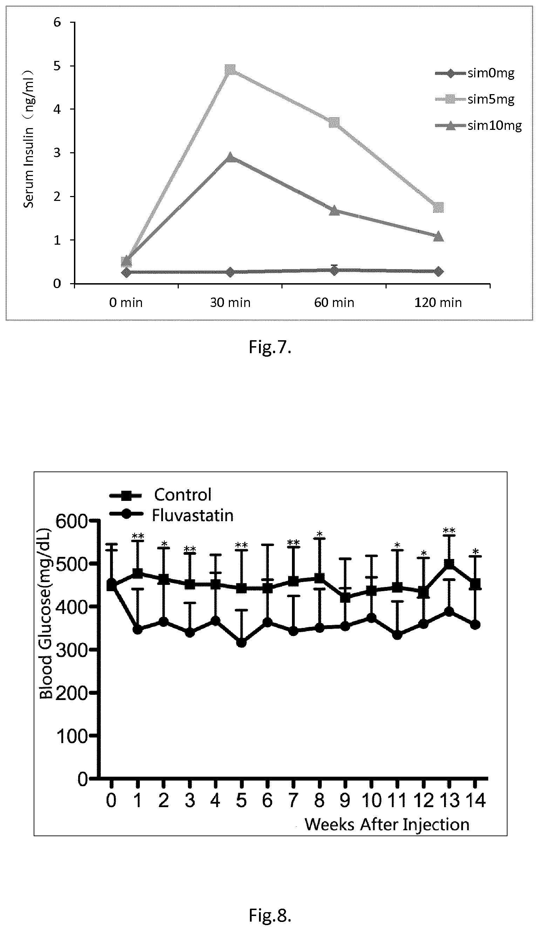

Figure 8 shows the fasting blood glucose changes 1-14weeks after a local, single injection of fluvastatin (see formula of example 3) into the femoral marrow cavity in diabetic rats. The fasting blood glucose was detected 1 to 14 weeks after a single intraosseous injection of fluvastatin into the diabetic rats' femoral marrow cavities. At the same time, blood was collected via the tail after 12 h of fasting, and a glucose meter measured the blood glucose changes. The results show that a single local intraosseous injection of fluvastatin significantly reduced fasting blood glucose, and this effect lasted at least 14 weeks.

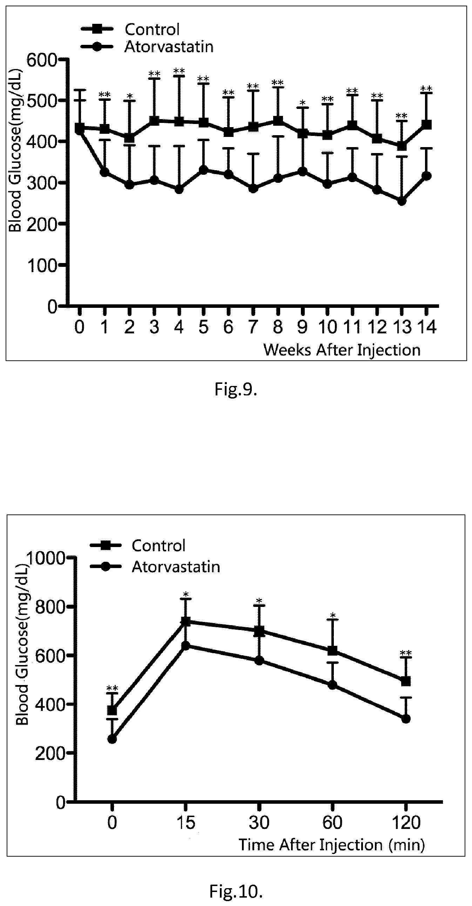

Figure 9 shows the fasting blood glucose changes 1-14 weeks after a local, single injection of atorvastatin (see formula of example 4) into the femoral marrow cavity in diabetic rats. The fasting blood glucose was detected 1 to 14 weeks after a single intraosseous injection of atorvastatin into the diabetic rats' femoral marrow cavities. At the same time, blood was collected via the tail after 12 h of fasting, and a glucose meter measured the blood glucose changes. The results show that a single local intraosseous injection of atorvastatin significantly reduced fasting blood glucose, and this effect lasted at least 14 weeks.
Figure 10 shows the result of a glucose tolerance test (GTT) 11 weeks after a local, single injection of atorvastatin (see formula of example 4) into the femoral marrow cavity in diabetic rats.
   According to the standard protocol, after fasting for 12h, the rats received an intraperitoneal injection of glucose (2g/kg), and a GTT test was conducted by blood glucose meter.

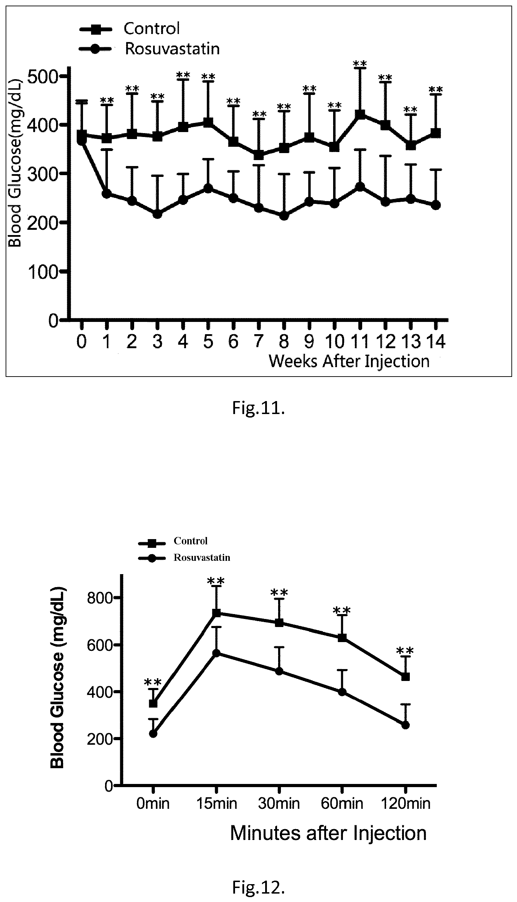

Figure 11 shows the fasting blood glucose changes 1-14 weeks after a local, single injection of rosuvastatin (see formula of example 5) into the femoral marrow cavity in diabetic rats. The fasting blood glucose was detected 1 to 14 weeks after a single intraosseous injection of rosuvastatin into the diabetic rats' femoral marrow cavities. At the same time, blood as collected via the tail after 12 h of fasting, and a glucose meter measured the blood glucose changes. The results show that a single local intraosseous injection of rosuvastatin significantly reduced fasting blood glucose, and this effect lasted at least 14 weeks.
Figure 12 shows the result of a glucose tolerance test (GTT) 11 weeks after a local, single injection of rosuvastatin (see formula of example 5) into the femoral marrow cavity in diabetic rats.
   According to the standard protocol, after fasting for 12h, the rats received an intraperitoneal injection of glucose (2g/kg), and a GTT test was conducted by blood glucose meter.
Figure 13 shows serum cholesterol changes 5 months after a single, local intraosseous injection of simvastatin in the STZ and high-fat diet induced type 2 diabetic rats (compared with the control group, ***p<0.001).
Figure 14 shows serum triglyceride changes 5 months after a single, local intraosseous injection of simvastatin in the STZ and high-fat diet induced type 2 diabetic rats (compared with the control group, ***p<0.05).
Figure 15 shows serum low density lipoprotein changes 5 months after a single, local intraosseous injection of simvastatin in the STZ and high-fat diet induced type 2 diabetic rats (compared with the control group, *p<0.05, **p<0.01).
Figure 16 shows alanine aminotransferase (ALT) changes 5 months after a single, local intraosseous injection of simvastatin in the STZ and high-fat diet induced type 2 diabetic rats (compared with the control group, ***p<0.001).
Figure 17 shows aspartate transaminase (AST) changes 5 months after a single, local intraosseous injection of simvastatin in the STZ and high-fat diet induced type 2 diabetic rats (compared with the control group, **p<0.01).
Figure 18 shows body weight changes 4 weeks after a single, local intraosseous injection of simvastatin in the high-fat diet induced obese rats (compared with the control group, **p<0.01).
Figure 19 shows the gonadal fat and body weight ratio 4 weeks after a single, local intraosseous injection of simvastatin in the high-fat diet induced obese rats (compared with the control group, *p<0.05).
Figure 20 shows MRI detection of body fat changes 4 weeks after a single local intraosseous injections of simvastatin in high-fat diet induced obese rats. The results shows that in the control group, the rats are obviously obese with increased peri-renal fat, while in the group with intraosseous injections of simvastatin, the body fat is significantly reduced.
Figure 21 shows that the fat liver significantly reduced 4 weeks after single local intraosseous injections of simvastatin in high-fat diet induced obese rats.
Figure 22 shows the systolic pressure changes 4 weeks after a single, local intraosseous injection of simvastatin in the high-fat diet induced obese rats (compared with the control group, *p<0.05, **p<0.01).
Figure 23 shows the diastolic pressure changes 4 weeks after a single, local intraosseous injection of simvastatin in the high-fat diet induced obese rats (compared with the control group, **p<0.01).

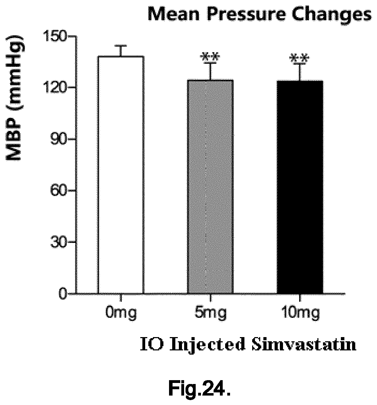

Figure 24 shows the mean blood pressure changes 4 weeks after a single, local intraosseous injection of simvastatin in the high-fat diet induced obese rats (compared with the control group, **p<0.01).

### Description of the preferred embodiments

The present invention is described in detail using examples given below, but the examples shall not be construed as a restriction to the scope of the present invention.

### The preparation of pharmaceutical composition

### Example 1

### Preparation of local injection of simvastatin

Simvastatin 1000mg was dissolved in PBS phosphate buffer 10ml (Beijing Zhongshan Golden Bridge Biotechnology Co., Ltd.), PBS solution containing 2% DMSO (Dimethyl Sulfoxide, DMSO, purchased from Sigma) and 0.1 % bovine serum albumin (Bovine Serum Albumin, BSA, purchased from Sigma), and the resulting solution was made homogeneous, then a composition was obtained.

### Example 2

### Preparation of local injection of provastatin

Pravastatin sodium 1000mg was suspended in lipid emulsion (Intralipid) 10ml and the resulting solution was homogenized.

### Example 3

### Preparation of local injection of fluvastatin

Fluvastatin sodium 1000mg was dissolved in 10ml physiological saline containing 3% Poloxamer 188 (Germany BSF Company), and a homogeneous solution was obtained.

### Example 4

### Preparation of local injection of atovastatin

Atorvastatin calcium 1000mg was dissolved in PBS phosphate buffer 10ml (Beijing Zhongshan Golden Bridge Biotechnology Co., Ltd.), PBS solution containing 2% DMSO (Dimethyl Sulfoxide, DMSO, purchased from Sigma) and 0.1 % bovine serum albumin (Bovine Serum Albumin, BSA, purchased from Sigma), and the resulting solution was a homogeneous solution that is convenient for injection.

### Example 5

### Preparation of local injection of rosuvastatin

Rosuvastatin calcium 1000mg was dissolved in 10ml physiological saline containing 3% Poloxamer 188 (Germany BSF Company), and a homogeneous solution was obtained.

### Example 6

### Preparation of local injection of simvastatin

Simvastatin sodium 1000mg was suspended in lipid emulsion (Intralipid) 10ml and the resulting solution was homogenized.

### Example 7

### Preparation of local injection of tenivastatin

Tenivastatin 8000mg was dissolved in PBS phosphate buffer 10ml (Beijing Zhongshan Golden Bridge Biotechnology Co., Ltd.), PBS solution containing 2% DMSO (Dimethyl Sulfoxide, DMSO, purchased from Sigma) and 0.1 % bovine serum albumin (Bovine Serum Albumin, BSA, purchased from Sigma), and the resulting solution was a homogeneous solution that is convenient for injection.

### Example 8

### Preparation of local injection of pitavastatin

Pitavastatin 200mg was dissolved in 10ml physiological saline containing 3% Poloxamer 188 (Germany BSF Company), and a homogeneous solution was obtained.

### Diabetes pharmacological experiment of pharmaceutical preparations

### The experimental example 1 (reference)

### A single dose of simvastatin intraosseous injection promotes insulin secretion in ovariectomized rats

In accordance with standard operating procedures, adult female rats were bilaterally ovariectomized. In short, three month old female SD rats (weighing 200-250g) were anesthetized (10% chloral hydrate intraperitoneal injection of anesthesia, 3ml/kg), the left and right sides of the back of the rats were incised, the ovaries were found in the fat tissue, and both ovaries were removed. After 3 months, the rats were randomly divided into three groups (control, simvastatin 5mg, simvastatin 10mg), n=12 for each group. Under anesthesia (10% chloral hydrate intraperitoneal injection, 3ml/kg) and sterile conditions, the rats were incised 0.5cm under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoided leakage and sutured the wounds.

One and five months later respectively, six rats were randomly selected from each group, and the rats were euthanasized by overdose anesthesia., Serum was collected, and a 1-125 labeled radioimmunoassay (1-125 labeled radioimmunoassay kit purchased from Beijing North biotechnology Institute) with BH6020 type modular counter (Beijing nuclear Instrument Factory) measured the insulin levels. Surprisingly, serum insulin levels were significantly increased in a dose-dependent manner in the rats that were intraosseously injected with simvastatin. A single intraosseous injection of simvastatin signifcantly increased insulin levels for at least five months. (Figure 1- insulin levels one month after local injection) (Figure 2 - insulin levels five month after local injection) .

### The experimental example 2 (reference)

### Diabetes mellitus rat model of intraosseous injection of simvastatin

36 three-month-old male SD rats (250-300g) rats were accomodated for one week , in accordance with standard procedures of the diabetic rat model, with intraperitoneal injection of streptozotocin (STZ, Sigma Company) 40mg / kg, and next day administration of a high fat diet (purchased from Institute of Zoology, recipe : 68.5% base stock, lard 20%, 10% sucrose, 1% cholesterol and 0.5% porcine bile salts).

Two months later, all the rats had abnormally elevated blood glucose (above 19.5mmol/L), indicating a successful model (Zhouguang Xing animal models of human disease replication methodology Shanghai Science and Technology Literature Press, 2008 : 167-168 SHI Xin animal models of human disease People's Health Press, 2008: 301-305). After the diabetic model was established, the experimental animals were randomly divided into three groups (control group , simvastatin 5mg group, simvastatin 10mg group), n=12 for each group. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoided leakeage and sutured the wounds.

### The experimental example 3 (reference)

### Fasting blood glucose assay

The fasting rats' blood glucose levels were detected weekly, 8 weeks after a local single intraosseous injection of simvastatin by means of the femoral medullary cavity. After the rats were fasted for 6 h, one drop of blood (about 50 ul) was taken from the rat tail and placed in the blood sugar test paper (Roche). 2 seconds later, fasting blood glucose was determined by a Roche glucose meter (ACCU-CHEK Performa). Results show that blood glucose was significantly reduced after a single local intraosseous injection of simvastatin by means of the femoral medullary cavity, at least continuing for 14 weeks (figure 3).

The rats were killed to take blood, and blood glucose levels were measured using a blood biochemical Analyzer. Results showed that blood glucose was significantly decreased (Figure 4).

### The experimental example 4 (reference)

### Glucose Tolerance Test (GTT)

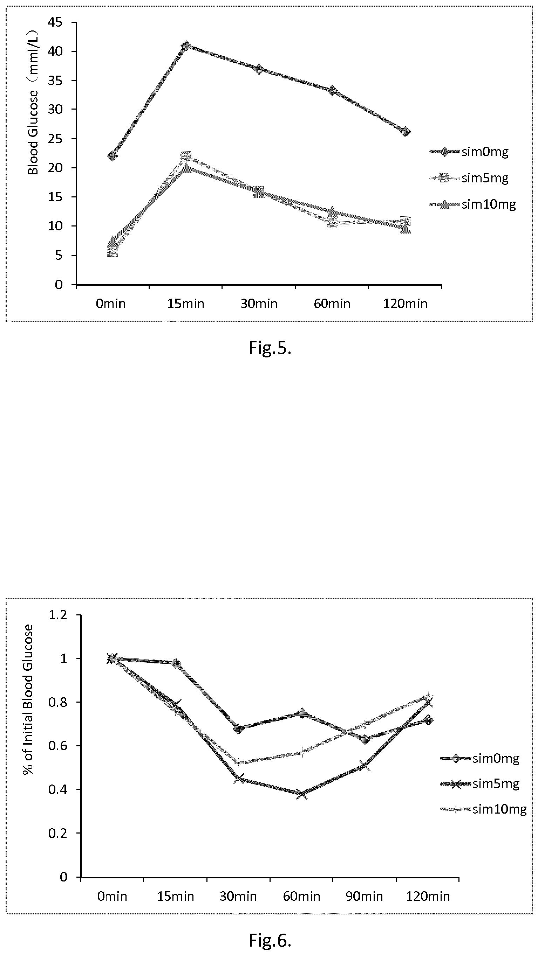

To further verify the anti-diabetic therapeutic effect of a single local intraosseous injection of simvastatin by means of the femoral medullary cavity, we conducted intraperitoneal injection of glucose tolerance tests. 11 weeks after intraosseous injection of simvastatin, the rats were overnight fasted for 12h. After intraperitoneal injection of glucose (2g/kg, China otsuka pharmaceutical co., LTD), and collecting blood from the tail vein 0, 15, 30, 60, 120 minutes later respectively, the blood glucose levels were assayed with a blood glucose meter (ACCU-CHEK Performa) blood glucose (Mauvais-Jarvis F, Ueki K, Fruman DA, et al. Reduced expression of the murine p85alpha subunit of phosphoinositide 3-kinase improves insulin signaling and ameliorates diabetes. J Clin Invest. 2002; 109(1): 141-149) (Results were show in Figure 5).

### The experimental example 5 (reference)

### Insulin Tolerance Test (ITT)

To further verify the anti-diabetic therapeutic effect of a single local intraosseous injection of simvastatin by means of the femoral medullary cavity, we conducted an insulin tolerance test. 11 weeks after intraosseous injection of simvastatin, rats were fasted for 6h. After intraperitoneal injection of neutral insulin (1U/kg, Wanbang pharmacy), we collected blood from the tail vein 0, 15, 30, 60, 90, 120 minutes later respectively, in the light of reported methods (Mauvais-Jarvis f, Ueki k, Fruman DA, et al. Reduced expression of the murine p85alpha subunit of phosphoinositide 3-kinase improves insulin signaling and ameliorates diabetes. J Clin Invest. 2002; 109:141-149.). Blood glucose levels were assayed with a Roche blood glucose meter (ACCU-CHEK Performa), and the ratio of initial blood glucose was calculated. Results show that single local intraosseous injection of simvastatin by means of the femoral medullary cavity could significantly improve insulin sensitivity (results shown in Figure 6.)

### The experimental example 6 (reference)

### Glucose-Stimulated Insulin Secretion Test (GSIS)

To further verify the anti-diabetic therapeutic effect of a single local intraosseous injection of simvastatin by means of the femoral medullary cavity, we conducted a glucose-stimulated insulin secretion test. 14 weeks after single local intraosseous injection of simvastatin, the rats were fasted for 12 h. After intraperitoneal injection of glucose (3g/kg, China otsuka pharmaceutical co., LTD.), at 0, 30, 60, 120 minutes we collected blood from the tail vein. In total, 200 ul of blood was collected from the rat's tail. The blood was left to stand at room temperature for 5 minutes, centrifuged at 4000rpm for 10 minutes, then about 20 ul was taken and serum rat trace insulin was detected with a serum rat trace insulin assay kit (Millipore Corp., Billerica, MA company) on the microplate reader (Thermo Fisher Scientific). Results are shown in figure 7.

### The experimental example 7 (reference)

### Diabetes mellitus rat model of intraosseous injection of fluvastatin

36 three-month-old male SD rats (250-300g) were accomodated for one week, in accordance with standard procedures of the diabetic rat model, with intraperitoneal injection of streptozotocin (STZ, Sigma Company) 40mg/kg, and next day administration of a high fat diet (purchased from Institute of Zoology, recipe : 68.5% base stock, lard 20%, 10% sucrose, 1% cholesterol and 0.5% porcine bile salts).

Two months later, all the rats had abnormally elevated blood glucose (above 19.5mmol/L), indicating a successful model (Zhouguang Xing animal models of human disease replication methodology Shanghai Science and Technology Literature Press, 2008 : 167-168 SHI Xin animal models of human disease People's Health Press, 2008: 301-305). After the diabetic model was established, the experimental animals were randomly divided into two groups (vehicle and fluvastatin 8mg), n=12 for each group. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a fluvastatin injection prepared according to Example 3, containing fluvastatin 8mg or only vehicle (3% poloxamer 188 normal saline) respectively, was injected with a micro syringe. Bone wax pinhole puncture closure avoided leakeage and sutured the wounds.

### Fasting blood glucose assay

The fasting rats' blood glucose levels were detected weekly, 1 week after local single intraosseous injection of fluvastatin by means of femoral medullary cavity. After the rats were fasted for 6 h, one drop of blood (about 50 ul) was taken from the rat tail and placed in the blood sugar test paper (Roche). 2 seconds later, fasting blood glucose levels were determined by a Roche glucose meter (ACCU-CHEK Performa). Results show that blood glucose significantly reduced after a single local intraosseous injection of fluvastatin, at least continuing for 14 weeks (figure 8).

### The experimental example 8 (reference)

### Diabetes mellitus rat model of intraosseous injection of atorvastatin by means of femoral medullary cavity

Thirty six 3-month-old male SD rats (250-300g) were accomodated for one week, in accordance with standard procedures of the diabetic rat model, with intraperitoneal injection of streptozotocin (STZ, Sigma Company) 40mg / kg, and next day administration of a high fat diet (purchased from Institute of Zoology, recipe : 68.5% base stock, lard 20%, 10% sucrose, 1% cholesterol and 0.5% porcine bile salts).

Two months later, all the rats had abnormally elevated blood glucose (above 19.5mmol/L), indicating a successful model (Zhouguang Xing. Animal models of human disease replication methodology Shanghai Science and Technology Literature Press, 2008 : 167-168 SHI Xin animal models of human disease People's Health Press, 2008: 301-305). After the diabetic model was established, the experimental animals were randomly divided into two groups (vehicle and atorvastatin 4mg), n=12 for each group. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and an atorvastatin injection prepared according to Example 4, containing atorvastatin 4mg or only vehicle (3% poloxamer 188 normal saline) respectively, was injected with a micro-syringe. Bone wax pinhole puncture closure avoided leakeage and sutured the wounds.

### Fasting blood glucose assay

The fasting glucose levels were detected weekly, 1 week after local single intraosseous injection of atorvastatin by means of the femoral medullary cavity. After the rats were fasted for 6 h, one drop of blood (about 50 ul) was taken from the rat tail and placed in the blood sugar test paper (Roche). 2 seconds later, fasting blood glucose levels were determined by a Roche glucose meter (ACCU-CHEK Performa) . Results show that blood glucose significantly reduced after a single local intraosseous injection of atorvastatin by means of the femoral medullary cavity, at least continuing for 14 weeks (figure 9).

### The experimental example 9 (reference)

### Glucose Tolerance Test (GTT)

To further verify the anti-diabetic therapeutic effect of a single local intraosseous injection of atorvastatin by means of the femoral medullary cavity, we conducted intraperitoneal injection of glucose tolerance tests. 11 weeks after intraosseous injection of atorvastatin, the rats were overnight fasted for 12h. After intraperitoneal injection of glucose (2g/kg, China otsuka pharmaceutical co., LTD), and collecting blood from the tail vein 0, 15, 30, 60, 120 minutes later respectively, the blood glucose levels were assayed with a blood glucose meter (ACCU-CHEK Performa) blood glucose (Mauvais-Jarvis F, Ueki K, Fruman DA, et al. Reduced expression of the murine p85alpha subunit of phosphoinositide 3-kinase improves insulin signaling and ameliorates diabetes. J Clin Invest. 2002; 109(1): 141-149). Results are shown in Figure 10.

### The experimental example 10 (reference)

### Diabetes mellitus rat model of intraosseous injection of rosuvastatin by means of femoral medullary cavity

Thirty-six 3-month-old male SD rats (250-300g) were accomodated for one week, in accordance with standard procedures of the diabetic rat model, with intraperitoneal injection of streptozotocin (STZ, Sigma Company) 40mg/kg, and next day administration of a high fat diet (purchased from Institute of Zoology, recipe : 68.5% base stock, lard 20%, 10% sucrose, 1% cholesterol and 0.5% porcine bile salts) .

Two months later, all the rats had abnormally elevated blood glucose (above 19.5mmol/L), indicating a successful model (Zhouguang Xing. Animal models of human disease replication methodology Shanghai Science and Technology Literature Press, 2008 : 167-168 SHI Xin animal models of human disease People's Health Press, 2008: 301-305). After the diabetic model was established, the experimental animals were randomly divided into two groups (vehicle and rosuvastatin 2mg), n=12 for each group. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a rosuvastatin injection prepared according to Example 5, containing rosuvastatin 2mg or only vehicle (3% poloxamer 188 normal saline) respectively, was injected with a micro-syringe. Bone wax pinhole puncture closure avoided leakeage and sutured the wounds.

### Fasting blood glucose assay

The fasting rats' blood glucose levels were detected weekly, 1 week after local single intraosseous injection of rosuvastatin by means of the femoral medullary cavity. After the rats were fasted for 6 h, one drop of blood (about 50 ul) was taken from the rat tail and placed in the blood sugar test paper (Roche). 2 seconds later, fasting blood glucose levels were determined by a Roche glucose meter (ACCU-CHEK Performa) . Results show that blood glucose significantly reduced after a single local intraosseous injection of rosuvastatin by means of the femoral medullary cavity, at least continuing for 14 weeks. Results are shown in Figure 11.

### The experimental example 11 (reference)

### Glucose Tolerance Test (GTT)

To further verify the anti-diabetic therapeutic effect of a single local intraosseous injection of rosuvastatin by means of the femoral medullary cavity, we conducted intraperitoneal injection of glucose tolerance tests. 11 weeks after intraosseous injection of rosuvastatin, the rats were overnight fasted for 12h. After intraperitoneal injection of glucose (2g/kg, China otsuka pharmaceutical co., LTD), and collecting blood from the tail vein 0, 15, 30, 60, 120 minutes later respectively, the blood glucose levels were assayed with a blood glucose meter (ACCU-CHEK Performa) blood glucose (Mauvais-Jarvis F, Ueki K, Fruman DA, et al. Reduced expression of the murine p85alpha subunit of phosphoinositide 3-kinase improves insulin signaling and ameliorates diabetes. J Clin Invest. 2002; 109(1): 141-149). Results are shown in Figure 12.

### The experimental example 12

### A single local intraosseous injection of simvastatin by means of femoral medullary cavity significantly decreases blood lipid in STZ and high fat diet fed rats

On the second day after the administration of an intraperitoneal injection of streptozotocin (STZ, Sigma) 40mg/kg, rats were given a high-fat diet (purchased from the Institute of Zoology, the formula : Basic materials 68.5% , 20% lard and sucrose 10 % , 1% cholesterol , 0.5% porcine bile salts).

The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoided leakeage and sutured the wounds.

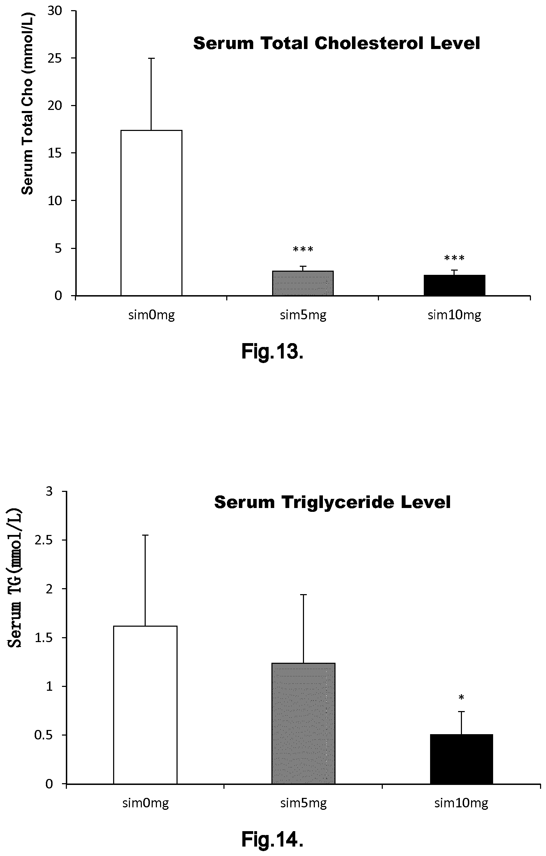

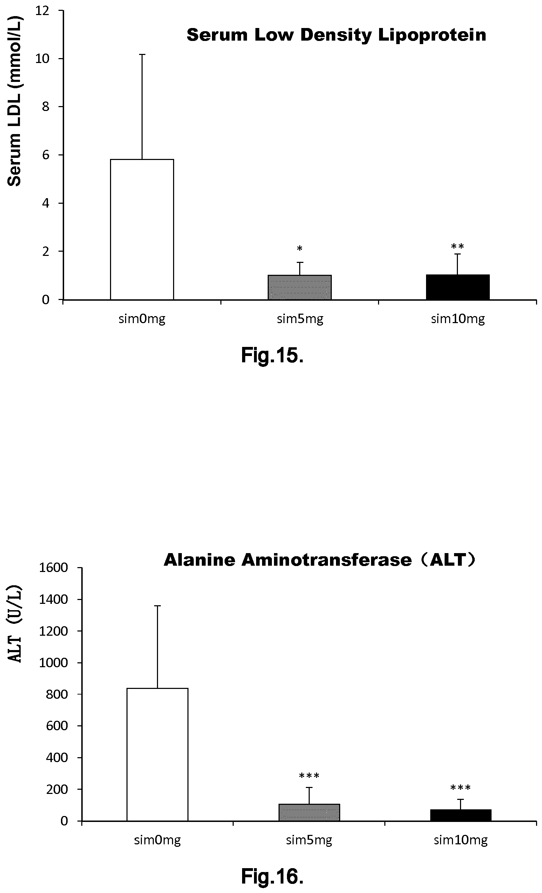

Five months later, the rats were euthanized by overdose anesthesia, the serum was collected, and serum cholesterol, triglycerides, low-density lipoprotein levels were detected. Results show a single intraosseous injection of simvastatin by means of the femoral medullary cavity significantly decreased serum cholesterol, triglycerides, and LDL even after 5 months (compared with the control group, *<0.05, **p<0.01, ***p<0.001). For results see Figure 13 (serum total cholesterol), Figure 14 (serum triglycerides), Figure 15 (serum low density lipoprotein).

### The experimental example 13

### A single local intraosseous injection of simvastatin by means of femoral medullary cavity significantly increases liver function in STZ and high fat diet fed rats

On the second day after the administration of an intraperitoneal injection of streptozotocin (STZ, Sigma) 40mg/kg, rats were given a high-fat diet (purchased from the Institute of Zoology, the formula: Basic materials 68.5% , 20% lard and sucrose 10 % , 1% cholesterol , 0.5% porcine bile salts).

The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoidied leakeage and sutured the wounds.

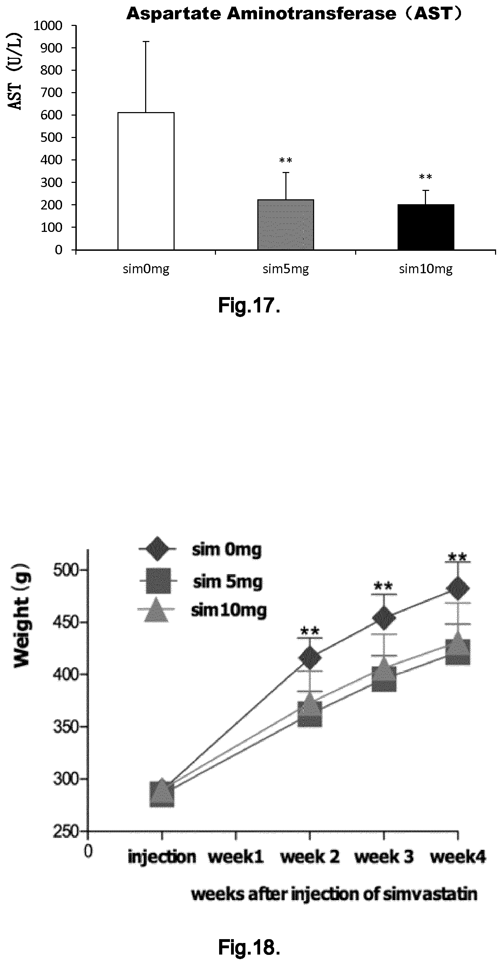

Five months later, the rats were euthanized by overdose anesthesia, the serum was collected, and alanine aminotransferase (ALT) and aspartate aminotransferase (AST) levels were detected. Results show a single intraosseous injection of simvastatin significantly improved liver function in rats. Results are shown in Figure 16 (ALT), Figure 17 (AST).

### The experimental example 14

### A single local intraosseous injection of simvastatin by means of femoral medullary cavity significantly reduces obesity in high fat diet fed rats

A high-fat diet (purchased from the Institute of Zoology, the formula: Basic materials 68.5%, 20% lard and sucrose 10 % , 1% cholesterol, 0.5% porcine bile salts) was given to rats. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a simvastatin injection prepared according to Example 1, simvastatin 5mg, simvastatin 10mg or only vehicle (PBS buffer, 0.1% BSA, 2% DMSO) respectively, was injected with a micro syringe. Bone wax pinhole puncture closure avoided leakeage and sutured the wounds.

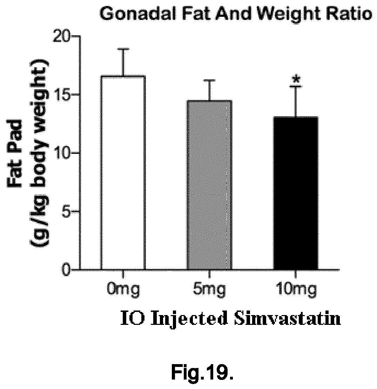

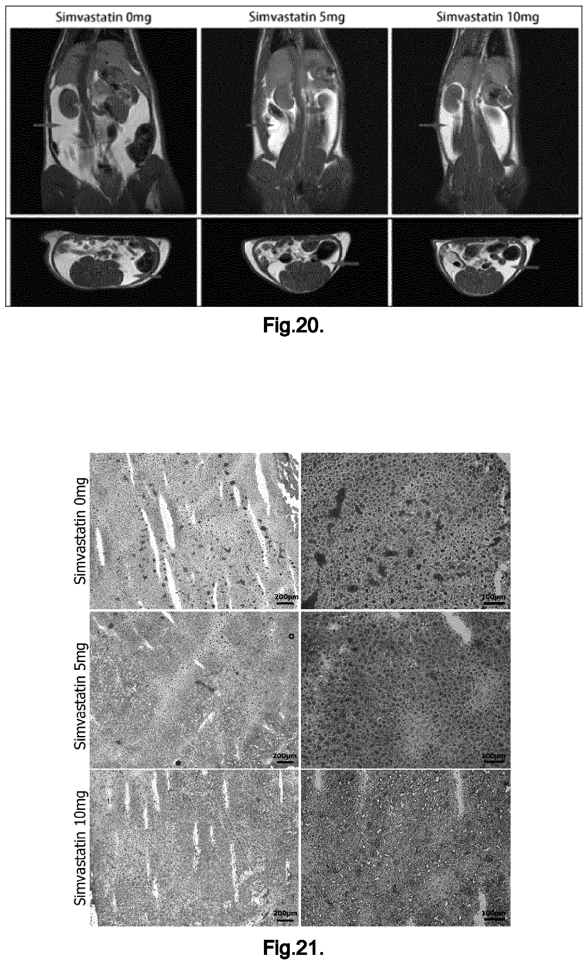

All the rats were weighed once a week after the operation, and 4 weeks later the rats were euthanized by overdose anesthesia. MRI detection of peri-renal fat and weighing of gonadal fat was performed, and the ratio of gonadal adipose tissue and body weight was calculated. Results show that a single local intraosseous injection of simvastatin significantly reduced body weight and fat volume. Figure 18 (body weight), Figure 19 (ratio of gonadal adipose tissue and body weight), Figure 20 (MRI detection of peri-renal fat).

### The experimental example 15

### A single local intraosseous injection of simvastatin by means of femoral medullary cavity significantly reduces fat liver

A high-fat diet (purchased from the Institute of Zoology, the formula: Basic materials 68.5%, 20% lard and sucrose 10 %, 1% cholesterol, 0.5% porcine bile salts) was given to rats. The rats were anesthetized by intraperitoneal injection of 10% chloral hydrate anesthesia (3ml/kg), incised 0.5cm under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoided leakeage and sutured the wounds.

All the rats were weighed once a week after the operation; the rats were euthanized by the overdose anesthesia 4 weeks later. Rat livers were frozen sectioned and stained with red oil, results show that fatty liver was significantly reduced. Results are as shown in Figure 21.

### The experimental example 16

### A single local intraosseous injection of simvastatin by means of femoral medullary cavity significantly reduces fat liver

A high-fat diet (purchased from the Institute of Zoology, the formula: Basic materials 68.5%, 20% lard and sucrose 10 %, 1% cholesterol, 0.5% porcine bile salts) was given to rats. The rats were anesthetized by intraperitoneal injection of 10% chloral hydrate anesthesia (3ml/kg), incised 0.5cm at a sterile environment under the right side of the greater trochanter in the lateral femur, a medullo-puncture needle (diameter 1mm) was used to penetrate the cortical bone, and a simvastatin injection prepared according to Example 1, containing simvastatin 5mg, simvastatin 10mg or only vehicle (PBS buffer, 0.1% BSA, 2% DMSO) respectively, was injected with a micro syringe. Bone wax pinhole puncture closure avoidied leakeage and sutured the wounds.

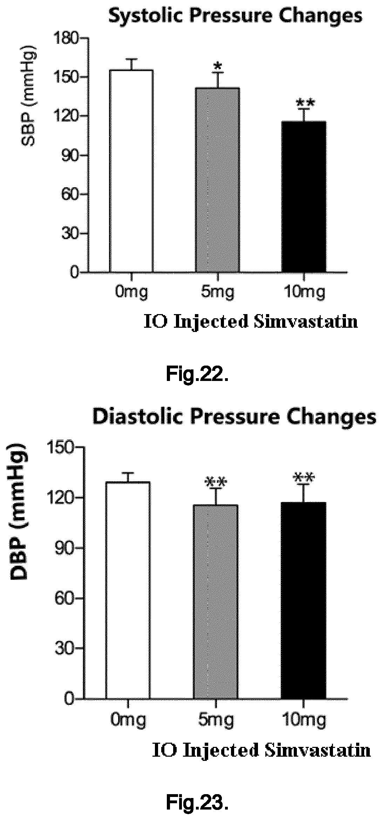

Weighed per week after surgery, rats were anesthetized after 3 weeks and blood pressure was measured; results show that blood pressure was significantly decreased in the experimental group. Results are shown in Figure 22 (systolic blood pressure), Figure 23 (diastolic), 24 (mean arterial pressure).

## Claims

1. A statin compound (HMG CoA reductase inhibitor), or its pharmaceutically acceptable salt or a pharmaceutical composition containing a statin compound and/or its pharmaceutically acceptable salt for use in:
(a) the prevention, by local single intraosseous administration, of obesity, hypertension, hyperlipidemia, atherosclerosis, or fatty liver;
(b) the treatment, by local single intraosseous administration, of obesity, hypertension, hyperlipidemia, atherosclerosis, cardiovascular or cerebrovascular diseases which are coronary heart disease or stroke, or fatty liver; or
(c) the therapeutic improvement, by local single intraosseous administration, of fat metabolism in mammals including human beings;
wherein the dosing interval of the administration is once every 7 days up to 600 days.

2. A compound, salt or composition for use according to claim 1, wherein local administration is administration into the bone, preferably within the bone with bone marrow cavity.

3. A compound, salt or composition for use according to claim 2, wherein the compound, salt or composition is provided in an injectable dosage form, said injectable dosage form being optionally selected from an injection solution, suspension, emulsion, gel, injectable solid form, or their sustained-release and controlled-release forms.

4. A compound, salt or composition for use according to any one of claims 1-3,
wherein the dosing interval of the administration is once every 10 to 500 days at a time, more preferably 20 to 400 days at a time, most preferably 30 to 300 days at a time.

5. A compound, salt or composition for use according to any one of claims 1-4,
wherein the dosage of statin compound ranges from 0.1mg to 1000mg, preferably from 1mg to 500mg, more preferably from 2mg to 200mg, most preferably from 2mg to lOmg.

6. A compound, salt or composition for use according to any one of claims 1-5, wherein the statin compound is selected from simvastatin, atorvastatin, fluvastatin, lovastatin, pravastatin, rosuvastatin, pitavastatin, bervastatin, cerivastatin, crilvastatin, dalvastatin, mevasatin and tenivastatin; preferably simvastatin, atorvastatin, fluvastatin and rosuvastatin; the most preferably simvastatin; and the pharmaceutically acceptable salt form is selected from hydrochloride, hydrobromide, hydriodate, sulfate, nitrate, phosphate, citrate, mesylate, trifluoroacetate, acetate, or salt of sodium, potassium, magnesium and calcium.

7. A compound, salt or composition for use according to any one of claims 1-6, wherein a pharmaceutically acceptable carrier, dilutent or excipient is included, said pharmaceutical acceptable carrier, diluent or excipient being selected from water-soluble solvent, oily solvent, dispersing agent, isotonic agent, preservative, solubilizer and stabilizers.

8. A compound, salt or composition for use according to claim 7 wherein the water-soluble solvent is selected from distilled water, normal saline, Ringer's solution and phosphate buffer (PBS); the oil soluble solvent is selected from vegetable oil, such as olive oil, castor oil, sesame oil, cottonseed oil or corn oil; the dispersing agent is selected from tween 20 and tween 80, polyethylene glycol, carboxy methyl cellulose, and/or sodium alginate; the isotonic agent is selected from sodium chloride, glycerol, sorbic alcohol, and glucose; the solubilizer is selected from salicylic acid sodium, poloxamer and sodium acetate; the preservative is selected from methyl paraben, propyl paraben, benzyl alcohol, chlorobutanol, sodium benzoate, and phenol; the stabilizer is selected from albumin, such as human serum albumin, and bovine serum albumin; and wherein said pharmaceutically acceptable carrier, diluent or excipient is optionally also selected from biodegradable materials, such as polylactide, poly-l-lactide-glycolide and polyaspartic acid.

## Patentansprüche

1. Statinverbindung (HMG-CoA-Reduktase-Hemmer) oder ihr pharmazeutisch annehmbares Salz oder eine pharmazeutische Zusammensetzung, die eine Statinverbindung enthält und/oder ihr pharmazeutisch annehmbares Salz zur Verwendung bei Folgendem:
(a) der Prävention, durch lokale, einzelne, intraossäre Verabreichung, von Fettleibigkeit, Bluthochdruck, Hyperlipidämie, Atherosklerose oder Fettleber;
(b) der Behandlung, durch lokale, einzelne, intraossäre Verabreichung, von Fettleibigkeit, Bluthochdruck, Hyperlipidämie, Atherosklerose, Herz-Kreislauf- oder zerebrovaskulären Erkrankungen, die koronare Herzkrankheit oder Schlaganfall sind, oder Fettleber;
(c) der therapeutischen Verbesserung, durch lokale, einzelne, intraossäre Verabreichung, von Fettstoffwechsel bei Sägetieren einschließlich Menschen;
wobei das Dosierungsintervall der Verabreichung einmal alle 7 Tage bis zu 600 Tage ist.

2. Verbindung, Salz oder Zusammensetzung zur Verwendung nach Anspruch 1, wobei lokale Verabreichung Verabreichung in den Knochen ist, bevorzugt in den Knochen mit Knochenmarkhohlraum.

3. Verbindung, Salz oder Zusammensetzung zur Verwendung nach Anspruch 2, wobei die Verbindung, das Salz oder die Zusammensetzung in einer injizierbaren Dosierungsform bereitgestellt ist, wobei die injizierbare Dosierungsform optional ausgewählt ist aus einer Injektionslösung, einer Suspension, einer Emulsion, einem Gel, einer injizierbaren festen Form oder den Formen mit verzögerter Freisetzung und mit kontrollierter Freisetzung.

4. Verbindung, Salz oder Zusammensetzung zur Verwendung nach einem der Ansprüche 1-3, wobei das Dosierungsintervall der Verabreichung jeweils einmal alle 10 bis 500 Tage ist, bevorzugt jeweils 20 bis 400 Tage, am bevorzugtesten jeweils 30 bis 300 Tage.

5. Verbindung, Salz oder Zusammensetzung zur Verwendung nach einem der Ansprüche 1-4, wobei die Dosierung von Statinverbindung von 0,1 mg bis 1000 mg reicht, bevorzugt von 1 mg bis 500 mg, bevorzugter von 2 mg bis 200 mg, am bevorzugtesten von 2 mg bis 10 mg.

6. Verbindung, Salz oder Zusammensetzung zur Verwendung nach einem der Ansprüche 1-5, wobei die Statinverbindung ausgewählt ist aus Simvastatin, Atorvastatin, Fluvastatin, Lovastatin, Pravastatin, Rosuvastatin, Pitavastatin, Bervastatin, Cerivastatin, Crilvastatin, Dalvastatin, Mevasatin und Tenivastatin; bevorzugt Simvastatin, Atorvastatin, Fluvastatin, und Rosuvastatin; am bevorzugtesten Simvastatin; und die pharmazeutisch annehmbare Salzform ausgewählt ist aus Hydrochlorid, Hydrobromid, Hydriodat, Sulfat, Nitrat, Phosphat, Citrat, Mesylat, Trifluoracetat, Acetat oder Salz von Natrium, Kalium, Magnesium und Calcium.

7. Verbindung, Salz oder Zusammensetzung zur Verwendung nach einem der Ansprüche 1-6, wobei ein pharmazeutisch annehmbarer Träger, ein Verdünnungsmittel oder ein Hilfsstoff beinhaltet ist, wobei der pharmazeutisch annehmbare Träger, das Verdünnungsmittel oder der Hilfsstoff ausgewählt ist aus wasserlöslichem Lösungsmittel, öligem Lösungsmittel Dispergiermittel, isotonischem Mittel, Konservierungsmittel, Lösungsvermittler und Stabilisatoren.

8. Verbindung, Salz oder Zusammensetzung zur Verwendung nach Anspruch 7, wobei das wasserlösliche Lösungsmittel ausgewählt ist aus destilliertem Wasser, isotonischer Kochsalzlösung, Ringerlösung und Phosphatpuffer (PBS); das öllösliche Lösungsmittel ausgewählt ist aus Pflanzenöl, wie z. B. Olivenöl, Rizinusöl, Sesamöl, Baumwollsamenöl oder Maisöl; das Dispergiermittel ausgewählt ist aus Tween 20 und Tween 80, Polyethylenglykol, Carboxymethylcellulose und/oder Natriumalginat; das isotonische Mittel ausgewählt ist aus Natriumchlorid, Glyzerin, Sorbinalkohol und Glukose; der Lösungsvermittler ausgewählt ist aus Salicylsäurenatrium, Poloxamer und Natriumacetat; das Konservierungsmittel ausgewählt ist aus Methylparaben, Propylparaben, Benzylalkohol, Chlorbutanol, Natriumbenzoat und Phenol; der Stabilisator ausgewählt ist aus Albumin, wie z. B. Humanserumalbumin und Rinderserumalbumin; und wobei der pharmazeutisch annehmbare Träger, das Verdünnungsmittel oder der Hilfsstoff optional auch ausgewählt ist aus biologisch abbaubaren Materialien, wie z. B. Polylactid, Poly-l-lactid-glycolid und Polyasparaginsäure.

## Revendications

1. Composé de statine (inhibiteur de l'HMG CoA réductase), ou son sel pharmaceutiquement acceptable ou une composition pharmaceutique contenant un composé de statine et / ou son sel pharmaceutiquement acceptable à utiliser dans :
(a) la prévention, par administration intra-osseuse locale unique, de l'obésité, l'hypertension, l'hyperlipidémie, l'athérosclérose ou la stéatose hépatique ;
(b) le traitement, par administration intra-osseuse locale unique, de l'obésité, de l'hypertension, de l'hyperlipidémie, de l'athérosclérose, des maladies cardiovasculaires ou cérébrovasculaires qui représente une maladie coronarienne ou un accident vasculaire cérébral, ou une stéatose hépatique ; ou
(c) l'amélioration thérapeutique, par administration intra-osseuse locale unique, du métabolisme des graisses chez les mammifères, y compris les humains ;
dans lequel l'intervalle de dosage de l'administration est une fois tous les 7 jours jusqu'à 600 jours.

2. Composé, sel ou composition à utiliser selon la revendication 1, dans lequel l'administration locale est une administration dans l'os, préférablement à l'intérieur de l'os avec une cavité médullaire.

3. Composé, sel ou composition à utiliser selon la revendication 2, dans lequel le composé, sel ou composition est fourni sous une forme galénique injectable, ladite forme galénique injectable étant éventuellement choisie parmi une solution injectable, une suspension, une émulsion, un gel, une forme de solide injectable, ou leurs formes à libération prolongée et à libération contrôlée.

4. Composé, sel ou composition à utiliser selon l'une quelconque des revendications 1 à 3, dans lequel l'intervalle de dosage de l'administration est une fois tous les 10 à 500 jours à la fois, plus préférablement 20 à 400 jours à la fois, le plus préférablement 30 à 300 jours à la fois.

5. Composé, sel ou composition à utiliser selon l'une quelconque des revendications 1 à 4, dans lequel le dosage du composé de statine est de 0,1 mg à 1000 mg, préférablement de 1 mg à 500 mg, plus préférablement de 2 mg à 200 mg, le plus préférablement de 2 mg à 10 mg.

6. Composé, sel ou composition à utiliser selon l'une quelconque des revendications 1 à 5, dans lequel le composé de statine est choisi parmi la simvastatine, l'atorvastatine, la fluvastatine, la lovastatine, la pravastatine, la rosuvastatine, la pitavastatine, la bervastatine, la cérivastatine, la crilvastatine, la dalvastatine, la mévasatine et la ténivastatine ; préférablement la simvastatine, l'atorvastatine, la fluvastatine et la rosuvastatine ; le plus préférablement la simvastatine ; et la forme de sel pharmaceutiquement acceptable est choisie parmi le chlorhydrate, le bromhydrate, l'hydriodate, le sulfate, le nitrate, le phosphate, le citrate, le mésylate, le trifluoroacétate, l'acétate ou le sel de sodium, potassium, magnésium et calcium.

7. Composé, sel ou composition à utiliser selon l'une quelconque des revendications 1 à 6, dans lequel un support, diluant ou excipient pharmaceutiquement acceptable est inclus, ledit support, diluant ou excipient pharmaceutiquement acceptable étant choisi parmi un solvant hydrosoluble, un solvant huileux, un agent dispersant, agent isotonique, un conservateur, un solubilisant et des stabilisants.

8. Composé, sel ou composition à utiliser selon la revendication 7, dans lequel le solvant hydrosoluble est choisi parmi l'eau distillée, la solution saline normale, la solution de Ringer et le tampon phosphate (PBS) ; le solvant soluble dans l'huile est choisi parmi l'huile végétale, telle que l'huile d'olive, l'huile de ricin, l'huile de sésame, l'huile de coton ou l'huile de maïs ; l'agent dispersant est choisi parmi le tween 20 et le tween 80, le polyéthylène glycol, la carboxyméthylcellulose et / ou l'alginate de sodium ; l'agent isotonique est choisi parmi le chlorure de sodium, le glycérol, l'alcool sorbique et le glucose ; le solubilisant est choisi parmi l'acide salicylique sodique, le poloxamère et l'acétate de sodium ; le conservateur est choisi parmi le méthyl paraben, le propyl paraben, l'alcool benzylique, le chlorobutanol, le benzoate de sodium et le phénol ; le stabilisant est choisi parmi l'albumine, telle que la sérumalbumine humaine, et la sérumalbumine bovine ; et dans lequel ledit support, diluant ou excipient pharmaceutiquement acceptable est éventuellement également choisi parmi des matériaux biodégradables, tels que le polylactide, le poly-1-lactide-glycolide et l'acide polyaspartique.
